**Europäisches Patentamt**

**European Patent Office**

**Office européen des brevets**

(19)

(11) Numéro de publication: **0 225 223**
**B1**

(12) # FASCICULE DE BREVET EUROPEEN

(45) Date de publication du fascicule du brevet:
**06.09.89**

(51) Int. Cl.⁴: **C 07 C 67/313,** C 07 C 69/716

(21) Numéro de dépôt: **86402361.9**

(22) Date de dépôt: **22.10.86**

(54) **Procédé de fabrication de glyoxylate d'alkyle.**

(30) Priorité: **23.10.85 FR 8515740**

(43) Date de publication de la demande:
**10.06.87 Bulletin 87/24**

(45) Mention de la délivrance du brevet:
**06.09.89 Bulletin 89/36**

(84) Etats contractants désignés:
**AT BE CH DE ES FR GB IT LI LU NL SE**

(56) Documents cités:
**EP-A- 0 130 533**
**EP-A- 0 149 456**

(73) Titulaire: **SOCIETE FRANCAISE HOECHST Société anonyme dite:, 3, avenue du Général de Gaulle, F-92800 Puteaux (FR)**

(72) Inventeur: **Plagne, Jean-Louis, 26 Avenue du Bordelet, F-30200 Bagnole-Sur-Ceze (FR)**
Inventeur: **Che, Michel, 21 Avenue Franklin Roosevelt, F-92330 Sceaux (FR)**
Inventeur: **Delafosse, Denise, 7 rue Maurice Couderchet, F-94120 Fontenay-Sous-Bois (FR)**

(74) Mandataire: **Santarelli, Marc, Cabinet Rinuy et Santarelli 14, avenue de la Grande Armée, F-75017 Paris (FR)**

## Description

La présente invention concerne un procédé de préparation d'un glyoxylate d'alkyle par déshydrogénate catalytique oxydante en phase gazeuse du glycolate d'alkyle correspondant.

Les glyoxylates d'alkyle sont des matières premières précieuses pour accéder notamment à l'allantoïne ou à des homopolymères (brevet des Etats-Unis d'Amérique N° 4 144 226).

La déshydrogénation catalytique oxydante en phase gazeuse d'un glycolate d'alkyle en glyoxylate correspondant est connue par les brevets des Etats-Unis d'Amérique N° 1 614 195; 4 340 748 et les demandes de brevets européens N° 0 130 533; 0 149 439 et 0 149 456 mettant en oeuvre différents catalyseurs.

C'est ainsi que selon les enseignements de l'état de la technique, cette déshydrogénation oxydante catalytique est réalisée soit à 200-250°C sur du vanadate d'argent ou sur du vanadate de cuivre (brevet des Etats-Unis d'Amérique N° 1 614 195), soit à une température comprise entre 100 et 600°C, de préférence entre 200 et 400°C sur un catalyseur contenant au moins un élément choisi dans le groupe constitué par l'argent, le cuivre, le molybdène et le vanadium et activé par au moins un élément choisi dans le groupe consitué par l'antimoine, le bismuth, l'étain, les métaux alcalins ou alcalino-terreux (brevet des Etats-Unis d'Amérique N° 4 340 748), soit à 400°C sur un catalyseur contenant au moins un élément choisi dans le groupe constitué par l'argent, le vanadium, le molybdène, le cuivre, l'or, l'étain, l'antimoine, le bismuth et le phosphore (demande de brevet européen N° 0 130 533), soit à une température comprise entre 325°C et 685°C sur de l'argent (demande de brevet européen N° 0 149 439), soit enfin dans un réacteur spécial (demande de brevet européen N° 0 149 456).

Or la Demanderesse a mis au point un procédé de préparation d'un glyoxylate d'alkyle par déshydrogénation catalytique oxydante en phase gazeuse du glycolate d'alkyle correpondant caractérisé par le fait qu'il est réalisé sur un catalyseur à base d'oxyde de vanadium V déposé sur un oxyde de titane IV.

L'oxyde de titane IV peut être de la forme rutile, anatase ou un mélange en proportions variables de ces deux formes allotropiques, mais avantageusement l'oxyde de titane IV utilisé est principalement de la forme anatase. Son aire spécifique peut varier dans de grandes proportions, de quelques mètres carrés à une centaine de mètres carrés par gramme. Les caractéristiques des oxydes de titane IV utilisés dans le procédé selon l'invention sont mentionnées dans le tableau I ci-après dans lequel l'aire spécifique, désignée AS, est donnée en mètres carrés par gramme et les taux de phase anatase et rutile en grammes pour cent grammes.

L'oxyde de titane IV, référence P 25, est commercialisé par la Société Degussa AG, Postfach 110533, 6000 Frankfurt am Main 11, République Fédérale d'Allemagne, l'oxyde de titane IV, anatase, par la Société Tioxyde SA, Boîte Postale 89, 62102 Calais, France, et l'oxyde de titane IV, référence Chalumeau, a été préparé selon la méthode décrite par M. Formenti et al., J. Colloïd Interface SCI., 1972, 39, 79.

*Tableau I*

| Références et origines | | AS | Taux de phase | |
|---|---|---|---|---|
| | | | Anatase | Rutile |
| T1 | P 25 | 50 | 68 | 32 |
| T2 | Anatase | 10 | >95 | < 5 |
| T3 | Rutile obtenu par calcination du P25 pendant 15 heures à 900°C sous air | 8 | 0 | 100 |
| T4 | Chalumeau | 91 | 60 | 40 |

Les taux pondéraux d'oxyde de vanadium V déeposé sur oxyde de titane IV peuvent varier dans de grandes proportions, de 0,1 à 90%, mais avantageusement ces taux sont choisis de manière à obtenir un recouvrement unimoléculaire d'oxyde de vanadium V sur l'oxyde de titane IV, ce qui correspond de 1 à 1,8 mg d'oxyde de vanadium V par mètre carré d'aire spécifique de l'oxyde de titane IV choisi.

Les dépôts d'oxyde de vanadium V sur le support oxyde de titane IV sont réalisés par diverses méthodes connues en soi telles que l'imprégnation classique, P1, la préparation au chalumeau, P2, la double imprégnation en milieu éthanol, P3, l'attaque de l'oxyde de vanadium V supporté en milieu ammoniacal, P4, ou le greffage par de l'oxytrichlorure de vanadium, P5.

L'imprégnation classique, P1, consiste à agiter pendant deux heures une suspension d'oxyde de titane IV dans une solution aqueuse de monovanadate d'ammonium et d'acide oxalique contenant la quantité de vanadium que l'on souhaite déposer puis à concentrer à sec cette suspension, à la sécher enquite quinze heures à 100°C et cinq heures à 450°C.

La préparation au chalumeau, P2, consiste à brûler dans la flamme d'un chalumeau oxhydrique des vapeurs de tétrachlorure de titane et de tétrachlorure de vanadium en proportions souhaitées.

L'attaque de l'oxyde de vanadium V en milieu ammonical, P3, est décrite par A. Miyamoto et al., J. Phys. Chem., 1981, 2366. Par cette méthode, on a préparé les catalyseurs mentionnés dans le tableau II ci-après dans lequel on précise les conditions opératoires: poids de l'échantillon préparé par imprégnation' classique avec un taux de 30% en poids d'oxyde de vanadium, molarité et poids en grammes de la solution aqueuse ammoniacale d'attaque et le taux pondéral d'oxyde de vanadium V résiduaire désigné T X V.

*Tableau II*

| Références | Conditions operatoires | | | |
|---|---|---|---|---|
| | Poids du précurseur | Solution d'attaque | | T x V |
| | | Molarité | Poids | |
| MA 1 | 2 | 0,3 | 300 | 2,9 |
| MA 2 | 2 | 0,1 | 100 | 3,5 |
| MA 3 | 2 | 0,05 | 200 | 8,6 |
| MA 4 | 2 | 0,01 | 100 | 18,2 |

La double imprégnation en milieu éthanol, P4, et la méthode par greffage de l'oxytrichlorure de vanadium, P5, sont décrites respectivement par A.J. Van Hengstum et al., Appl. Catal., 1983, 5, 207 et G.C. Bond et al., Faraday Discuss, 1981, 72, 235.

Les caractéristiques des différents catalyseurs préparés selon les méthodes décrites précédemment sont mentionnés dans le tableau III ci-après dans lequel sont précisés le taux pondéral en oxyde de vanadium V, désigné T X V, l'aire spécifique du catalyseur en mètres carrés par gramme, désignée AS, le poids d'oxyde de vanadium V en milligrammes par mètres carrés, désigné PdV et le nombre de monocouches théoriques d'oxyde de vanadium V, désigné NMT.

*Tableau III*

| Référence | Référence du Support | Méthode de Preparation | T x V | Aire Specifique | PdV | NMT |
|---|---|---|---|---|---|---|
| I1 | T1 | P1 | 2,2 | 47 | 0,47 | 0,35 |
| I2 | T1 | P1 | 3,1 | 46,5 | 0,67 | 0,45 |
| I3 | T1 | P1 | 6,3 | 46 | 1,37 | 0.95 |
| I4 | T1 | P1 | 12,2 | 45 | 2,7 | 1,9 |
| I5 | T1 | P1 | 30 | 20 | 15 | 10,5 |
| IA | T2 | P1 | 1,25 | 10 | 1,25 | 0,9 |
| IR | T3 | P1 | 1,2 | 8 | 1,5 | 1.04 |
| IC | T4 | P1 | 13,8 | 85 | 1,62 | 1,1 |
| C1 | - | P2 | 3,6 | 77 | 0,47 | 0,35 |
| C2 | - | P2 | 10,7 | 41,5 | 2,58 | 1,8 |
| C3 | - | P2 | 17,7 | 46 | 3,85 | 2,7 |
| C4 | - | P2 | 80,8 | 40 | 20,2 | 14,4 |
| MA1 | T1 | P4 | 2,9 | 49 | 0,59 | 0,4 |
| MA2 | T1 | P4 | 3,5 | 46 | 0,76 | 0,55 |
| MA3 | T1 | P4 | 8.6 | 45 | 1,91 | 1,3 |
| MA4 | T1 | P4 | 18,1 | 40 | 4,52 | 3,2 |
| MI | T1 | P3 | 2,2 | 46 | 0,48 | 0,35 |
| MG | T1 | P5 | 5,9 | 44 | 1,34 | 0,95 |

Selon le procédé de l'invéntion, le glycolate d'alkyle engagé est un ester de l'acide glycolique avec un alcool de formule générale ROH dans laquelle R représente un radical alkyle aliphatique, linéaire ou ramifié, contenant de 1 à 8 atomes de carbone, de préférence de 1 à 4. Il est mis en oeuvre à l'état de vapeur, diluée ou non avec un gaz interte tel que l'azote, un gaz rare.

On fait passer sur le catalyseur décrit précédement, disposé à l'état de lit fluidisé ou non dans un réacteur de configuration adéquate pour ce type de réaction, un mélange gazeux, généralement préchauffé à une température légèrement infériere à la température réactionelle, constitué d'oxygène et de vapeur du glycolate d'alkyle choisi soit seuls, soit, et cela est préférable, accompagnés d'un gaz vecteur inerte dans les conditions de réactions choisies.

On peut utiliser comme source d'oxygène et de gaz vecteur, l'air.

Selon le procédé de l'invention, on engage avantageusement par mole de glycolate d'alkyle:

1 à 100 moles, de préférence de 20 à 80 moles d'un gaz inerte,

de 0,1 à 10 moles, de préférence de 1 à 5 moles d'oxygène ou la quantité correspondante d'un gaz oxygène tel que l'air.

Le catalyseur selon le procédé de l'invention est avantageusement mélangé avec une charge telle que la ponce, un siliciure de carbone, un charbon.

Le procédé selon l'invention est mis en oeuvre à une température supérieure ou égale à 150°C. Les temps de séjour sont compris avantageusement entre 0,1 et 10 secondes, de préférece entre 0,1 et 5 secondes.

Le procédé selon l'invention est habituellement effectué à la pression atmosphérique bien qu'il soit possible d'opérer sous des pressions plus faibles ou plus élevées, de 0,01 à 100 bars.

On a constaté avec surprise que l'énergie d'activation apparente de l'oxydation déshydrogénante des glycolates d'alkyle est faible avec le système catalytique de l'invention. C'est ainsi que cette énergie est voisine de 75 Kj par mole pour le glycolate de méthyle; cette faible valeur traduit bien l'aptitude du système catalytique à activer le glycolate de méthyle.

L'activité et la sélectivité des catalyseurs selon le procédé de l'invention appliqués à la déshydrogénation oxydante catalytique du glycolate de méthyle, en glyoxylate de méthyle, (désigné GxMe), sont, données dans le tableau IV dans lequel on mentionne: la référence du catalyseur; son taux pondéral en oxyde de vanadium V, (T X V); son aire spécifique en mètres carrés par gamme déterminée par la méthode B.E.T. décrite dans le brevet français N° 593 470 (désigné AS); le nombre de monocouches théoriques d'oxyde de vanadium V (désigné NMT); a vitesse intrinsèque de disparition du glycolate de méthyle en moles par seconde et par mètre carré multipliées par un milliard (désignée VI).

*Tableau IV*

| Ref. | T x V | AS | NMT | VI | SI |
|------|-------|------|------|------|------|
| I1   | 2,2   | 47   | 0,35 | 8,5  | 76 |
| I2   | 3,1   | 46,5 | 0,45 | 13,3 | 84 |
| I3   | 6,3   | 46   | 0,95 | 17,2 | 88 |
| I4   | 12,2  | 45   | 1,90 | 13,5 | 88 |
| IA1  | 1,25  | 10   | 0,9  | 22   | 88 |
| IC   | 13,8  | 85   | 1,1  | 12,5 | 81 |
| C1   | 3,6   | 77   | 0,35 | 3,6  | 79 |
| C2   | 10,7  | 41,5 | 1,8  | 17,3 | 88 |
| C3   | 17,7  | 46   | 2,7  | 7,0  | 84 |
| MA1  | 2,9   | 49   | 0,4  | 10,1 | 83 |
| MA2  | 3,5   | 46   | 0,55 | 18   | 87 |
| MA3  | 8.6   | 45   | 1,3  | 17,5 | 86 |
| MA4  | 18,1  | 40   | 3,2  | 11,3 | 85 |
| MI   | 2,2   | 46   | 0,35 | 12,7 | 83 |
| MG   | 5,9   | 44   | 0,95 | 18,4 | 91 |

Les essais furent affecutés à 150°C et la pression atmosphérique sur une masse de catalyseur de 50 mg, avec un débit de 1 à 2 litres par heure d'un mélange gazeux constitué en pourcentages molaires de 94% d'hélium, 3,3% d'oxygène et 2,7% de glycolate de méthyle soit pour une mole de glycolate de méthyle, 1,22 mole d'oxygène et 35 moles d'hélium.

La sélectivité initiale, SI, exprime la sélectivité de la réaction pour un temps de contact nul, c'est-à-dire que cette valeur représente la pente à l'origine de la courbe du taux de transformation molaire en glyoxylate d'alkyle mis en oeuvre (TTP) en fonction du taux de transformation global molaire (TTG) du glycolate d'alkyle engagé:

$$TTG = \frac{\text{Nombre de moles de réactif transformé}}{\text{Nombre de moles de réactif mis en oeuvre}} \times 100$$

$$TTP = \frac{\text{Nombre de moles de réactif transformé en produit P}}{\text{Nombre de moles de réactif mis en oeuvre}} \times 100$$

Sélectivité pour un produit P:

$$SP = \frac{TTP}{TTG} \times 100$$

Le tableau V mentionne les résultats acquis dans la déshydrogénation catalytique oxydante du glycolate de méthyle sous les conditions opératoires utilisées dans les essais dans le tableau IV mais avec un débit de 0,2 litre par heure au lieu de 1 à 2 litres par heure et avec 200 mg de catalyseur.

*Tableau V*

| TTP | Référence du catalyseur | I3 | C2 | MG | MA3 | I4 |
|------|------|------|------|------|------|------|
| GxMe | | 45,6 | 53,4 | 45 | 48,2 | 45,5 |
| Dioxyde de carbone | | 14,1 | 11,1 | 13,1 | 7,3 | 8,2 |
| Méthanol | | 1,5 | 0 | 3,1 | 0 | 1,0 |
| Formiate de méthyle | | 2,6 | 2 | 5,4 | 1 | 1,5 |
| TTG | | 96,6 | 98,5 | 96,9 | 91,5 | 76,8 |

Selon une variante du procédé selon l'invention, le catalyseur est dopé avec de l'oxyde d'argent. Les quantités d'oxyde d'argent déposées sur le catalyseur peuvent varier dans une grande proportion mais avantageusement le taux pondéral d'oxyde d'argent déposé est compris entre 0,1 et 5%.

Ce dopage s'effectue aisément en imprégnant le catalyseur avec une solution aquese contenant la quantité souhaitée d'un sel d'argent soluble dans l'eau tel que le nitrate d'argent suivi du séchage et de la calcination sous air durant cinq heures à 450°C de la suspension obtenue.

Le tableau VI montre l'influence d'ajouts d'oxyde d'argent sur les propriétés catalytiques du catalyseur de la présente invention dans la déshydrogénation, oxydante du glycolate de méthyle réalisée à 150°C et à la pression atmospérique sur 50 mg de catalyseur I3 d'une surface spécifique de 46 mètres carrés par gramme et présentant un taux pondéral en oxyde de vanadium V de 6,3% et avec un débit de 1 à 2 litres par heure d'un mélange gazeux constitué, en proportions molaires, de 94% d'hélium, 3,3% d'oxygène et 2,7% de glycolate de méthyle.

*Tableau VI*

| Références | Taux pondéral d'argent | VI | SI |
|-----------|------------------------|----|----|
| DA 1 | 0,3% | 20 | 86 |
| DA 2 | 2,6% | 23 | 89 |

VI et SI conservent la signification donnée dans le tableau IV.

Pour un taux de transformation global de 83%, on constate que le catalyseur DA 2 permet d'obtenir en sortie de réacteur un mélange gazeux contenant 56% de glyoxylate de méthyle, 13% de dioxyde de carbone, 17% de glycolate de méthyle, 4% de formiate de méthyle et 2% d'oxalate de diméthyle.

Toutes les analyses ont été réalisées par chromatographie en phase gazeuse sur des colonnes de 2 mètres et d'un diamètre de 3 mm chargées avec soit un polymère styrènedivinylbenzène («Chromosorb 101»), soit avec un polyéthylèneglycol 20 000 déposé sur kieselguhr. Les prises d'essais sont effectuées en sortie de réacteur sur une boucle d'injection de 0,3 ml. Les surfaces des pics sont mesurées par un intégrateur.

Les exemples qui suivent illustrent la présente invention sans toutfois la limiter.

*Exemple 1*

Dans un réacteur classique d'oxydation déshydrogénante catalytique de laboratoire, placé dans un four thermostaté à 150°C, on place 700 mg d'une poudre fine, de 0,1 à 0,5 mm de diamètre, constituée par un mélange intime de 200 mg du catalyseur 13 et de 500 mg de silicure de carbone.

On alimente ensuite en continu le réacteur, maintenu à 150°C, avec un mélange gazeux, préchauffé à 120°C, constitué en proportions molaires de 94% d'hélium, 3,3% d'oxygène de 2,7% de glycolate de méthyle et sous un débit de 0,5 litre par heure. Après trois heures de mise en régime, la réaction est stabilisée et on recueille en sortie de réacteur un mélange gazeux qui est immédiatement analysé qualitativement et quantitativement par chromatographie en phase gazeuse. On obtient ainsi un mélange gazeux contenant en pourcentages molaires par rapport à cent moles de glycolate de méthyle mis en oeuvre, 52,5% de glyoxylate de méthyle, 18% de glycolate de méthyle, 11% de dioxyde de carbone, 5,5% d'oxalate de diméthyle et 2,3% de formiate de méthyle.

*Exemple 2*

On opère comme dans l'exemple 1 mais on augmente le débit gazeux à un litre par heure. On obtient ainsi en sortie du réacteur un mélange gazeux contenant en pourcentages molaires par rapport à cent moles de glycolate de méthlyle mis en oeuvre, 50% de glycolate de méthyle, 40% de glyoxylate de méthyle, 5,5% de dioxyde de carbone et 1% d'oxalate de diméthyle.

*Exemple 3*

Dans le réacteur décrit à l'exemple 1 et maintenu à 150°C, on place 50 mg du catalyseur DA2 à l'état de poudre fine, puis on l'alimente en continu avec un mélange gazeux préchauffé à 120°C et constitué en proportions molaires de 94% d'hélium, 3,3% d'oxygène et 2,7% de glycolate de méthyle avec un débit de 1,5 litre par heure. Après trois heures de mise en régime, la réaction se stabilise et on recueille en sortie de réacteur un mélange gazeux contenant en proportions molaires par rapport à cent moles de glycolate de méthyle mis en oeuvre, 50% de glycolate de méthyle, 39% de glyoxylate de méthyle, 6% de dioxyde de carbone et 1% d'oxalate de diméthyle.

Il va de soi que la présente invention n'a été décrite qu'à titre purement explicatif et nullement limitatif et que toute modification pourra y être apportée notamment au niveau des équivalences, sans toutefois sortir de son cadre.

**Revendications**

1. Procédé de préparation d'un glyoxylate d'alkyle en $C_1$-$C_8$ par déshydrogénation catalytique en phase gazeuse du glycolate d'alkyle correspondant, caractérisé en ce qu'il est réalisé sur un catalyseur contenant pondéralement de 0,1 à 90% d'oxyde de vanadium V déposé sur un oxyde de titane IV.

2. Procédé selon la revendication 1, caractérisé en ce que l'oxyde de titane utilisé est principalement de la forme anatase.

3. Procédé selon l'une quelconque des revendications 1 ou 2, caractérisé en ce que l'oxyde de vanadium V est déposé sur l'oxyde de titane IV à l'état d'une couche unimoléculaire.

4. Procédé selon l'une quelconque des revendications 1 à 3, caractérisé en ce que le catalyseur à base d'oxyde de vanadium V déposé sur un oxyde de titane IV est dopé avec de l'oxyde d'argent.

5. Procédé selon l'une quelconque des revendications 1 à 4, caractérisé en ce qu'il est mis en oeuvre au départ du glycolate de méthyle.

## Patentansprüche

1. Verfahren zur Herstellung eines C$_1$-C$_8$-Alkyl-glyoxylats durch katalytische Dehyrierung des entsprechenden Alkylglykolats in Gasphase, dadurch gekennzeichnet, daß es auf einem Katalysator durchgeführt wird, der 0,1 bis 90 Gew.% Vanadium(V)-oxid enthält, das auf einem Titan(IV)-oxid niedergeschlagen ist.

2. Verfahren nach Anspruch 1, dadurch gekennzeichnet, daß das verwendete Titanoxid hauptsächlich in Form von Anatas ist.

3. Verfahren nach einem Ansprüche 1 oder 2, dadurch gekennzeichnet, daß das Vanadium(V)-oxid auf dem Titan(IV)-oxid in Form einer monomolekularen Schicht niedergeschlagen ist.

4. Verfahren nach einem der Ansprüche 1 bis 3, dadurch gekennzeichnet, daß der Katalysator auf Basis von Vanadium(V)-oxid, das auf dem Titan(IV)-oxid niedergeschlagen ist, mit Silberoxid dotiert ist.

5. Verfahren nach einem Ansprüche 1 bis 4, dadurch gekennzeichnet, daß es ausgehend von Methylglykolat durchgeführt wird.

## Claims

1. Process for preparation of a C$_1$-C$_8$ alkyl glyoxylate by catalytic dehydrogenation in the gaseous phase of the corresponding alkyl glycolate, characterised in that the dehydrogenation is carried out with a catalyst containing 0.1 to 90% by weight vanadium (V) oxide deposited on titanium (IV) oxide.

2. Process as claimed in claim 1, characterised in that the titanium oxide used is principally in the anatase form.

3. Process as claimed in any one of claims 1 or 2, characterised in that the vanadium (V) oxide is deposited on tatanium (IV) oxide in the form of a monomolecular film.

4. Process as claimed in any one of claims 1 or 3, characterised in that silver oxide is added to the catalyst based on vanadium (V) oxide deposited on titanium (IV) oxide.

5. Process as claimed in any one of claims 1 to 4, characterised in that the starting alkyl glycolate is methyl glycolate.